# EUROPEAN PATENT APPLICATION

(11) **EP 3 524 238 A1**
(43) Date of publication of application: **14.08.2019**
(21) Application number: 19161186.2
(22) Date of filing: 16.12.2014
(51) Int. Cl.: A61K 31/167, A61P 27/02, A61K 45/06

(54) **CETP MODULATOR FOR USE IN THE TREATMENT OF EYE DISEASE**

(30) Priority: 19.12.2013 EP 13198668
(62) Divisional of application: 14812502.4
(71) Applicant: F. Hoffmann-La Roche AG, 4070 Basel (CH)
(72) Inventor: CHESNÉ, Evelyne, 68510 Sierentz (FR); NIESOR, Eric J., 1260 Nyon (CH)
(74) Representative: Schirlin, Julien

(57) **Abstract**

The present invention relates CETP modulator which is useful in the prevention, treatment, delaying progression and /or reduction of eye diseases.

## Description

The present invention relates CETP modulators which are useful in the prevention, treatment, delaying progression and /or reduction of eye diseases such as cataract, corneal clouding (opacification), glaucoma, uveitis, intraocular neovascular diseases, in particular proliferative retinopathies, Choroidal Neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD and its method of preventing, retarding and ameliorating eye disease, such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD..

In another embodiment, the invention comprises a CETP modulator for use as therapeutic active substances for the prevention, the treatment, the prophylaxis and /or reduction of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In a particular embodiment, the present invention comprises a pharmaceutical composition comprising the CETP modulator, in particular 5-[2-([[1-(2-ethylbutyt)cyclohexyl]carbonyl]amino)phenyl] 2-methylpropanethioate, and crospovidone (in particular crospovidone micronized), useful for the prevention, treatment, delaying progression, and/or reduction of eye diseases, such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD..

In another embodiment of the present invention relates to composition comprising a CETP modulator, in association with one or more carotenoid(s), in particular wherein the carotenoids are selected from xanthophylls, more particular wherein one carotenoid is lutein, most particularly wherein the carotenoids are lutein and one stereoisomer of zeaxanthin (more particularly zeaxanthin), useful for the prevention, treatment, delaying progression, and/or reduction of eye diseases, such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD. Said composition is a nutraceutical composition or a pharmaceutical composition useful in particular for the prevention, treatment, delaying progression, and/or reduction eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In a particular embodiment according to the invention the eyes diseases are cataract, corneal clouding (opacification), age-related macular degeneration (AMD), in particular AMD.

### Brief description of the figures:

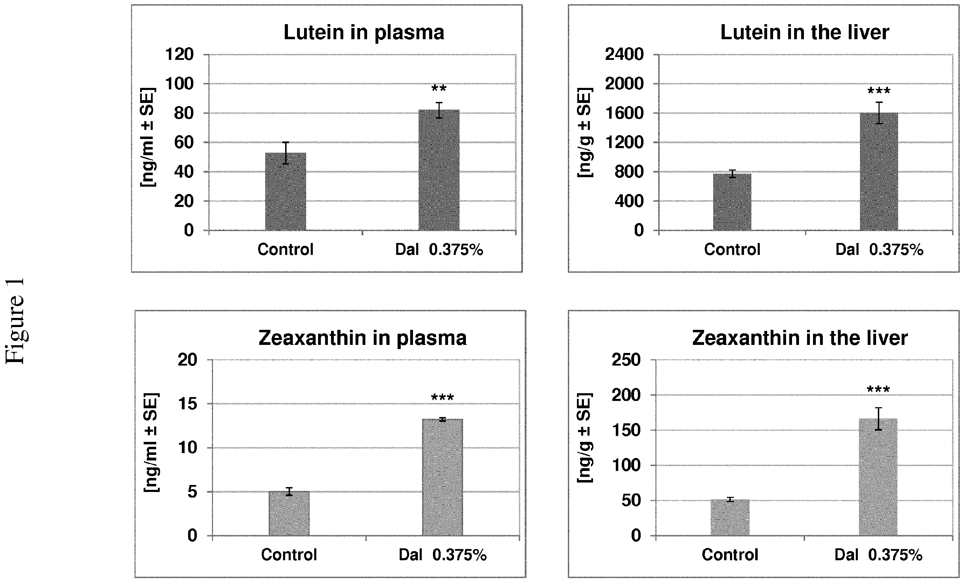

Figure 1: Effect of Dalcetrapib (Dal) on plasma & liver lutein & zeaxanthin levels in hamsters fed FLORAGLO study a.

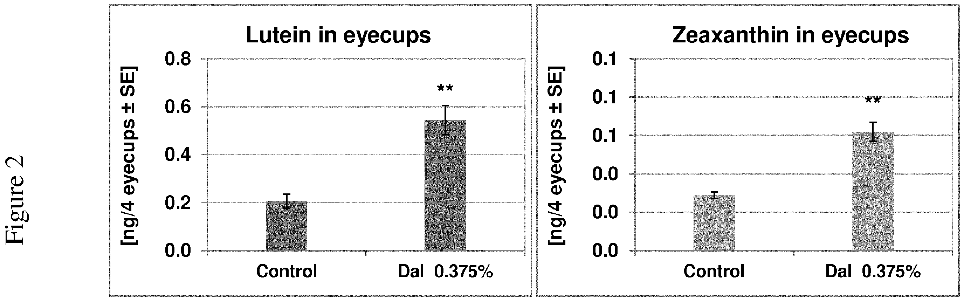

Figure 2: Effect of Dalcetrapib (Dal) on eyecups lutein & zeaxanthin levels in hamsters fed FLORAGLO study a.

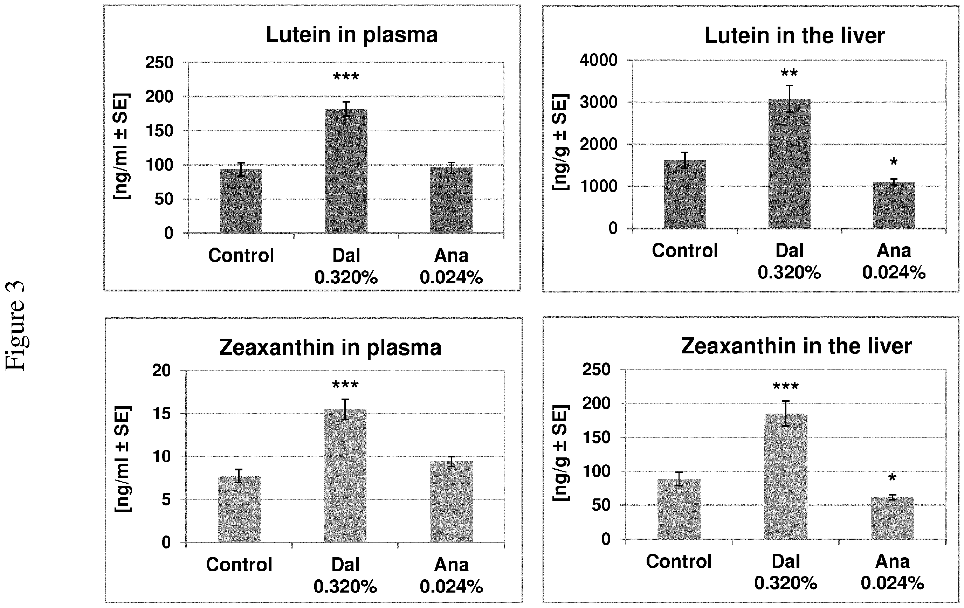

Figure 3: Effect of Dalcetrapib (Dal) & Anacetrapib (Ana) on plasma & liver lutein & zeaxanthin levels in hamsters fed FLORAGLO study b.

The carotenoids are naturally-occurring compounds that have antioxidant properties. The carotenoids are common compounds synthetized by plants, and contribute greatly to the colouring of plants and some animals. The large majority of animal species of including mammals, are unable to synthesize carotenoids *de novo* and accordingly rely upon diet to provide carotenoid requirements. Mammals also have a limited ability to modify carotenoids. A mammal can convert beta-carotene to vitamin A, but most other carotenoids are deposited in mammalian tissue in unchanged form. With respect to humans, about ten carotenoids are found in human serum. The major carotenoids in human serum are beta-carotene, alpha-carotene, cryptoxanthin, lycopene and lutein. Small amounts of zeaxanthin, phytofluene, and phytoene are found in human organs.

Plasma lycopene, lutein and beta-carotene are the most powerful antioxidants for explaining the concentration of oxidatively modified LDL in human serum (Karppi J et al. Atherosclerosis 2010 April;209(2):565). In the participants in the observational Atherosclerosis Risk in Communities (ARIC) study cohort patients were selected on the basis of B-mode carotid artery ultrasonograms were 231 asymptomatic age-, sex-, race-, and field center-matched case-control pairs. After adjustment for potential confounders, only the inverse association of lutein plus zeaxanthin with asymptomatic atherosclerosis was maintained. This study supports a modest inverse association between circulating levels of some carotenoids, particularly lutein plus zeaxanthin, and carotid intima-media thickness (IMT) (Iribarren C et al. Arterioscler Thromb Vasc Biol 1997 June;17(6):1171). Dwyer et al Dwyer et al, Circulation 2001, 103(24), 2922) assessed the protective effects of the oxygenated carotenoid on progression of IMT of the common carotid arteries determined ultrasonographically and concluded that increased dietary intake of lutein is protective against the development of early atherosclerosis. These findings suggest that these carotenoid compounds may be important in early stages of atherosclerosis. Pre-incubation of human aortic endothelial cells with beta-carotene, lutein and lycopene significantly reduced VCAM-1 expression by 29, 28, and 13%, respectively. Pre-incubation with beta-carotene and lutein significantly reduced E-selectin expression by 38 and 34%, respectively. Pre-treatment with beta-carotene, lutein and lycopene significantly reduced the expression of ICAM-1 by 11, 14, and 18%, respectively (Martin KR et al, Atherosclerosis 2000 June;150(2):265). Patients with CVD tend to have lower plasma levels of carotenoids (Lidebjer C et al, Nutr Metab Cardiovasc Dis 2007 July; 17(6):448).

Low levels of antioxidant carotenoids have been observed in subjects with mild cognitive impairment and with Alzheimer's disease by Rinaldi et al (Rinaldi P et al Neurobiol Aging 2003 November;24(7):915). Indeed, Xanthophyll such as lutein and zeaxanthin can cross the blood-retina barrier to preferentially accumulate in the macular region of the neural retina. They also accumulate in brain tissue. Recently Vishwanathan correlated the retinal levels of xynthophylls with those measured in the cerebellum and frontal cortex (Vishwanathan R et al, Nutr Neurosci 2013, 16(1), 21-).

Of the ten carotenoids found in human serum, only two, trans- and/or meso-zeaxanthin and lutein, have been found in the human retina. Zeaxanthin is the predominant carotenoid in the central macula or foveal region and is concentrated in the cone cells in the centre of the retina, i.e., the fovea. Lutein is predominantly located in the peripheral retina in the rod cells. Therefore, the eye preferentially assimilates zeaxanthin over lutein in the central macula which is a more effective singlet oxygen scavenger than lutein. It has been theorized that zeaxanthin and lutein are concentrated in the retina because of their ability to quench singlet oxygen and scavenge free radicals, and thereby limit or prevent photic damage to the retina.

It is known that the carotenoids other than zeaxanthin and lutein that do enter the retina may cause adverse effects, such as the formation of crystalline deposits by canthaxanthin, which may take several years to dissolve. Canthaxanthin in the retina also causes a decreased adaptation to the dark.

Vitamins (A, E or C), zinc, selenium, anthocyanins or macular pigments, such as, for example, lutein and zeaxanthin are usually used for their antioxidant properties. Lutein and zeaxanthin are macular pigments representing 99% of total pigments of the macula, which belong to the family of carotenoids, and specifically to the family of xanthophylls. Both pigments may exhibit on one hand an indirect protective antioxidant effect through their capacity to absorb blue light particularly aggressive for photoreceptor and on the other hand direct radical scavenging antioxidant properties. US2005/0032914, US2010/0159029 and WO2009/129859 describe compositions for oral administration, comprising lutein and zeaxanthin in combination with other non-enzymatic antioxidant products, for enhancing visual performance, inhibiting macular degeneration or promoting eye health. US2007 /265351 describes a xanthophyll composition comprising lutein and zeaxanthin useful for eye health.

Moreover, lutein is defined by the Afssa (Agence Francaise de Securite Sanitaire des Aliments) as "an agent which contributes to protect retina and lens against oxidation" (Saisine n°2003-SA-0205, 2004) and scientific studies have shown a relationship between the dietary intake of lutein/zeaxanthin and the likelihood of having AMD (SanGiovanni et al., AREDS report n°22, Arch Ophthalmol, 2007).

Recently, it has been demonstrated that aging eyes have a decreased amount of carotenoids deposited on the foveal region of the retina. Clinical and laboratory studies indicate that photic injury is at least one cause of age-related macular degeneration because of the cumulative effect of repeated photic insult which leads to a gradual loss of photoreceptor cells and degeneration of macular tissue.

Age-related macular degeneration (AMD) is an irreversible blinding disease of the retina. Unlike cataracts which can be restored by replacing of the diseased lens, age-related macular degeneration cannot be treated by replacing the diseased retina because the retina is a component of the central nervous system. Therefore, because no treatment for this disease exists once the photoreceptors are destroyed, prevention is the most efficient way to address age-related macular degeneration. Presently, prevention of age-related macular degeneration resides in limiting or preventing light and oxygen-induced (i.e., free radical-induced) damage to the retina because the retina is the only organ that is continuously exposed to high levels of light in a highly-oxygenated environment.

AMD is a leading cause of severe, irreversible vision loss among the elderly (Bressler, JAMA 291:1900-1 (2004)). It is characterized by a broad spectrum of clinical and pathologic findings, such as pale yellow spots known as drusen, disruption of the retinal pigment epithelium (RPE), choroidal neovascularization (CNV), and disciform macular degeneration. The manifestations of the disease are classified into two forms: non exudative (dry) and exudative (wet or neovascular). Drusen are the characteristic lesions of the dry form, and neovascularization characterizes the wet form. Disciform AMD is the fibrotic stage of the neovascular lesion.

There is a dramatic increase in the prevalence of AMD with advancing age. See, e.g. Leibowitz et al., Surv Ophthalmol 24(Suppl):335-610 (1980) and Klein et al., Ophthalmology 99:933-43 (1992). Although the wet form of AMD is much less common, it is responsible for 80%-90% of the severe visual loss associated with AMD (Ferris et al., Arch Ophthamol 102:1640-2 (1984)). There is an estimated 1-1.2 million prevalent cases of wet AMD. The cause of AMD is unknown; however, it is clear that the risk of developing AMD increases with advancing age. Other known risk factors include family history and cigarette smoking. Postulated risk factors also include oxidative stress, diabetes, alcohol intake, and sunlight exposure. D'Amico, N Engl J Med 331:95-106 (1994) and Christen et al., JAMA 276:1147-51 (1996).

Dry AMD is characterized by changes in the RPE and Bruch's membrane. It is thought that the RPE, compromised by age and other risk factors, deposits lipofuscin and cellular debris on Bruch's membrane. These changes may be seen ophthalmoscopically as drusen, which are scattered throughout the macula and posterior retinal pole. There are also variable degrees of atrophy and pigmentation of the RPE. Dry AMD may be asymptomatic or accompanied by variable and usually minimal visual loss and is considered to be a prelude to development of wet AMD.

Wet AMD is typically characterized by CNV of the macular region. The choroidal capillaries proliferate and penetrate Bruch's membrane to reach the RPE and may extend into the subretinal space. The increased permeability of the newly formed capillaries leads to accumulation of serous fluid or blood under the RPE and/or the neurosensory retina or within the neurosensory retina. When the fovea becomes swollen or detached, decreases in vision occur. Fibrous metaplasia and organization may ensue, resulting in an elevated subretinal mass called a disciform scar that constitutes end-stage AMD and is associated with permanent vision loss (D'Amico DJ. N Engl J Med 331:95-106 (1994)). The neovascularization in AMD can be classified into different patterns based on fluorescein angiography of subfoveal chorodial neovascular lesions. TAP and VIP Study Groups, Arch Ophthalmol 121:1253-68 (2003). The major angiographic patterns are termed classic and occult and are associated with different degrees of aggressiveness, vision losses, and response to different treatment options.

The group of xanthophylls includes (among many other compounds) lutein, zeaxanthin, neoxanthin, violaxanthin, and α- and β-cryptoxanthin. The latter compound is the only known xanthophyll to contain a beta-ionone ring, and thus β-cryptoxanthin is the only xanthophyll that is known to possess pro-vitamin A activity for mammals. Even then, it is a vitamin only for plant-eating mammals that possess the enzyme to make retinal from carotenoids that contain beta-ionone (some carnivores lack this enzyme). In species other than mammals, certain xanthophylls may be converted to hydroxylated retinal-analogues that function directly in vision. For example, with the exception of certain flies, most insects use the xanthophyll derived R-isomer of 3-hydroxyretinal for visual activities, which means that β-cryptoxanthin and other xanthophylls (such as lutein and zeaxanthin) may function as forms of visual "vitamin A" for them, while carotenes (such as beta carotene) do not.

In humans according to Kaplan et al. (Clin Physiol Biochem 1990, 8, 1) and Schmitz et al. (J Nutr 1991, 121, 1613) lutein ad zeaxanthin are accumulated in lipophilic tissues and are carried out by the lipoproteins.

Intestinal absorption and tissue distribution of carotenoids. Most likely because of their lipophilicity the absorption and distribution of carotenoids is complex and highly variable (Castenmiller, J.J. Annu Rev Nutr 1998;18:19). It involves intestinal components bile acids, enzymes such as esterase, receptors in enterocytes and inclusion into lipoproteins, secretion in the plasma or lymph and distribution among lipoproteins and delivery to tissues. Greene et al (Greene, C.M. Nutr Metab (Lond) 2006;3:-6) noticed a relationship between lutein and zeaxanthin plasma concentration and HDL size and speculated that because of their location at the surface of lipoproteins these carotenoids could exchange during particle remodeling while HDL interact with other lipoproteins. In addition, genetic variation is involved in the inter-individual variability in carotenoid bioavailability as reviewed by Borel (Borel P, Mol Nutr Food Res 2012 February;56(2):228) who suggests a personalized dietary guideline for carotenoids according to individual genetic characteristics. Several attempts to increase the bioavailability of lutein with various formulations have been published. Although the availability from egg seems satisfactory the simultaneous increase in plasma cholesterol may not be appropriate in a number of patients (Thurnham, D.I. Nutr Res Rev 2007 December;20(2):163).

Specific role of ABCA1 and pre-beta HDL in intestinal absorption. A first animal model of HDL deficiency syndrome has been described in chicken having a Z-linked mutation (Poernama, F. J Lipid Res 1990 June;31(6):955) later on found to affect ABCA1 function. Attie et al (Attie, A.D. J Lipid Res 2002 October;43(10):1610} identified the E89K mutation in the ABCA1 gene of the WHAM chicken and this animal model provided the direct demonstration that ABCA1 regulates efflux of cholesterol from the basolateral surface of the enterocyte to pre-beta/poorly lipidated ApoA1 (Mulligan,J.D. J Biol Chem 2003 April 11;278(15):13356). The role of pre-beta HDL is very likely the rate limiting step in xanthophyll uptake at the intestinal level since injection of mature HDL does not compensate for the absence of native poorly lipidated apoA1 (Mulligan, J.D. J Biol Chem 2003 April 11;278(15):13356). We hypothesized that HDL efficiently remodeled into pre-beta HDL by CETP activity (Niesor, E.J. 2010 J Lipid Res 2010 December;51(12):3443) is involved in the process of intestinal lipidation of native HDL by enterocyte and that trace amounts of sterols of plant origin (phytosterols) are also taken-up by this process (Niesor, E.J. Atherosclerosis 2011 December;219(2):761). We have previously shown that CETP is a major component of HDL remodeling and pre-beta-HDL formation in human plasma and that CETP modulators such as Dalcetrapib can enhance CETP activity involved in HDL remodeling (Niesor, E.J. 2010 J Lipid Res 2010 December; 51(12):3443). This will lead to an increase in pre-beta HDL formation in plasma, lipidation by the basolateral ABCA1 of enterocytes and eventually increase carotenoid absorption. This phenomenon was not observed with non-selective/complete CETP inhibitors such as torcetrapib. The same increase in pre-beta HDL occurring at the retinal level may increase the efflux of excess cholesterol and oxidized lipids as described by Ishida et al (Ishida, B.Y. Br J Ophthalmol 2006 May;90(5):616).

It has been found according to the present invention that not all CETP inhibitors are useful in increasing plasma and tissue xanthophylls for the prevention, treatment and or reduction delaying progression and /or reduction eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD. CETP modulator such as S-[2-[1-(2-ethylbutyl)cyclohexylcarbonylamino]-phenyl]2-methylthiopropionateare useful in the prevention, treatment and/or reduction of eye diseases in particularly AMD..

It was indeed proven that CETP inhibitors from two different chemical classes have been developed and reached late stage clinical development. The first class are potent CETP inhibitors which are 3,5-bis-trifluoromethyl-benzene derivatives such as torcetrapib (Clark RW, . Arterioscler Thromb Vasc Biol. 2004;24(3):490-7) and anacetrapib that have been shown to raise plasma HDL-C by up to 130% in humans ((Krishna R, Clin Pharmacol Ther. 2008 Dec;84(6):679-83.)). The phase III mortality/morbidity study of torcetrapib was terminated prematurely due to increased CV and non-CV deaths and events, due to off-target effects (Barter PJ, N Engl J Med. 2007;357(21):2109-22) unrelated to CETP inhibition (Forrest MJ, Br J Pharmacol. 2008; 154(7): 1465-73., Hu X, Endocrinology. 2009; 150(5):2211-9,). The second chemical class are "CETP modulator" such as benzenethiol derivatives, represented by S-[2-[1-(2-ethylbutyl)cyclohexylcarbonylamino]-phenyl] 2-methylthiopropionate which raises HDL-C by up to 36% in humans at the dose of 600mg (Stein EA, Am J Cardiol. 2009;104(1):82-91.) and was stopped in a phase III outcome study due to inefficacy for CVD patient.

In addition to potency, the mechanism of CETP inhibition by compounds derived from the two chemical classes is also likely to be different. The binding of torcetrapib to CETP increases its affinity for lipoproteins and induces the formation of an inactive high affinity complex between CETP and lipoproteins such as HDL (Clark RW, J Lipid Res 2006;47: 537-552). The CETP-lipoprotein complex, similar to the one induced by the CETP inhibitor antibody TP2 (Swenson TL, J Biol Chem 1989; 264:14318-26.) cannot efficiently exchange neutral lipid between different lipoprotein particles. Although the exact mechanism by which the CETP modulator such as S-[2-[1-(2-ethylbutyl)cyclohexylcarbonylamino]-phenyl] 2-methylthiopropionate decreases CETP activity has not been elucidated, the mode of action has been suggested to involve the direct specific interaction between cysteine 13 (Cys13) of CETP and benzenethiol moiety of 1-(2-Ethyl-butyl)-cyclohexanecarboxylic acid (2-mercapto-phenyl)-amide the active metabolite of S-[2-[1-(2-ethylbutyl)cyclohexylcarbonylamino]-phenyl] 2-methylthiopropionate, which is cleaved by non specific esterases in the gastrointestinal tract and in plasma, thus allowing the formation of a disulphide bound with CETP (Okamoto H, Nature 2000; 406(6792):203-207).

It is of interest to distinguish between the two classes of compounds as it appears that they have different mechanisms of action. Therefore there is a need to differentiate in vitro or ex vivo the CETP modulator such as S-[2-[1-(2-ethylbutyl)cyclohexylcarbonylamino]-phenyl] 2-methylthiopropionate, bis [2-[1-(2-ethylbutyl)cyclohexylcarbonylamino]phenyl] disulfide or 1-(2-Ethyl-butyl)-cyclohexanecarboxylic acid (2-mercapto-phenyl)-amide and its prodrug derivatives from all other CETP inhibitors, especially the potent CETP inhibitors such as 3,5-bis-trifluoromethyl-benzene derivatives (i.e. torcetrapib and anacetrapib).

A CETP modulator is thus defined as a compound which decreases cholesteryl ester transfer activity of CETP AND increases CETP induced HDL remodelling leading to an enhanced level of pre-beta/nascent HDL particles (Niesor JLR and Maugeais et al BBA Lipids).

Unless otherwise stated, the following terms used in the specification and claims have the meanings given below:
As used herein, the singulars forms "a", "an", and "the" include plural referents unless the contents clearly dictates otherwise. Therefore a compound optionally includes a combination of two or more such compound, and the like.

"CETP modulator " refers to a compound which modulates CETP activity (assessed by standard transfer assays) by inducing conformational changes of the CETP polypeptide once bound to the CETP polypeptide, wherein the conformational change is measured as hereunder described. Such specific interactions allow cholesteryl ester transfer activity of CETP to proceed between HDL particles AND increase CETP induced production of nascent pre-beta HDL. Preferably the CETP modulator refers to all compounds that would bind to cysteine 13 of the CETP polypeptide. More preferably, the "CETP modulator" is selected from S-[2-[1-(2-ethylbutyl)cyclohexylcarbonylamino]-phenyl] 2-methylthiopropionate, 1-(2-Ethyl-butyl)-cyclohexanecarboxylic acid (2-mercapto-phenyl)-amide and or bis [2-[1-(2-ethylbutyl)cyclohexylcarbonylamino]phenyl] disulfide. Most preferably, "CETP modulator" is S-[2-[1-(2-ethylbutyl)cyclohexylcarbonylaminol-phenyl]2-methylthiopropionate as a prodrug or 1-(2-Ethyl-butyl)-cyclohexanecarboxylic acid (2-mercapto-phenyl)-amide as its active metabolite.

The term "diluent" refers to an excipient which fills out the size of a tablet or capsule, making it practical to produce and convenient for the consumer to use. Suitable diluents include e.g. pharmaceutically acceptable fillers, such as microcrystalline cellulose (e.g. Avicel®), crospovidone micronized, cellulose powder, lactose spray-dried, lactose anhydrous, lactose monohydrate, dibasic calcium phosphate, sugars, sugar alcohols, corn starch, starch, pregelatinized starch, colloidal silicon dioxide, polysaccharides, and mixtures thereof.

Lutein is synthesized by plants and found in high quantities in green leafy vegetables such as spinach and kale. Lutein chemical structure is : 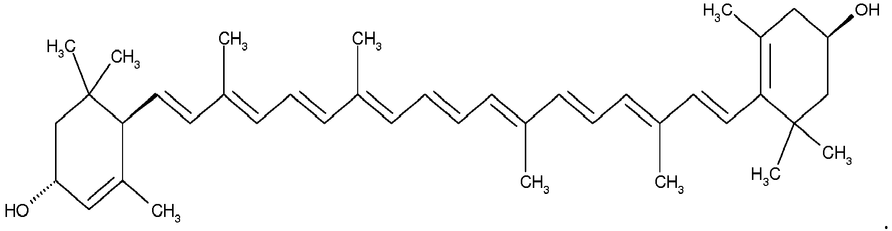 Lutein (CAS registry number 127-40-2) is also known as (3*R*,3'*R*,6'*R*)-β,ε-Carotene-3,3'-diol,

Zeaxanthin (CAS registry number 144-68-3) also known as (3*R*,3'*R*)-β,β-Carotene-3,3'-diol has the following chemical structure: 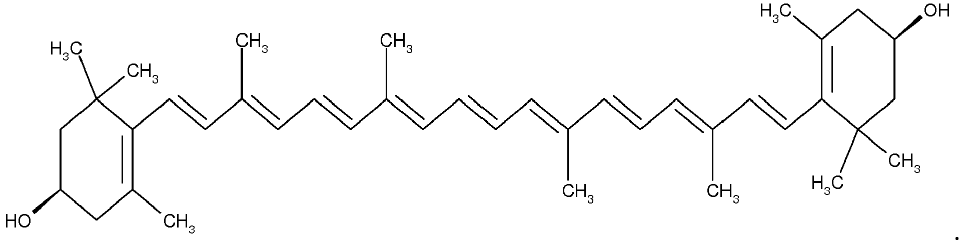 Zeaxanthin is present in corn, saffron, wolfberries.

The term "effective amount" or "therapeutically effective amount" refers to an amount of a drug effective to treat a disease or disorder in a mammal. In the case of age-related macular degeneration (AMD), the effective amount of the drug can reduce or prevent vision loss. For AMD therapy, efficacy in vivo can, for example, be measured by one or more of the following: assessing the mean change in the best corrected visual acuity (BCVA) from baseline to a desired time, assessing the proportion of subjects who lose fewer than 15 letters in visual acuity at a desired time compared with baseline, assessing the proportion of subjects who gain greater than or equal to 15 letters in visual acuity at a desired time compared with baseline, assessing the proportion of subjects with a visual-acuity Snellen equivalent of 20/2000 or worse at desired time, assessing the NEI Visual Functioning Questionnaire, assessing the size of CNV and amount of leakage of CNV at a desired time, as assessed by fluorescein angiography, etc. A "therapeutic dose" is a dose which exhibits a therapeutic effect on the patient and a subtherapeutic dose is a dose which does not exhibit a therapeutic effect on the patient treated.

An "intraocular neovascular disease" is a disease characterized by ocular neovascularization. Examples of intraocular neovascular diseases include, but are not limited to, e.g., proliferative retinopathies, choroidal neovascularization (CNV), age-related macular degeneration (AMD), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, etc.

The term "antibody" encompasses the various forms of antibody structures including but not being limited to whole antibodies and antibody fragments. The antibody according to the invention is preferably a humanized antibody, chimeric antibody, or further genetically engineered antibody as long as the antibody binds to an epitope of the CETP polypeptide wherein the said epitope is only accessible (exposed) to the antibody either in the CETP:CETP modulator complex form or in the CETP native form.

"Antibody fragments" comprise a portion of a full length antibody, preferably the variable domain thereof, or at least the antigen binding site thereof. Examples of antibody fragments include diabodies, single-chain antibody molecules, and multispecific antibodies formed from antibody fragments. scFv antibodies are, e.g. described in Houston, J.S., Methods in Enzymol. 203 (1991) 46-96).

The terms "monoclonal antibody" as used herein refer to a preparation of antibody molecules of a single amino acid composition.

The term "epitope" includes any polypeptide determinant capable of specific binding to an antibody. In certain embodiments, epitope determinant includes chemically active surface groupings of molecules such as amino acids, sugar side chains, phosphoryl, or sulfonyl, and, in certain embodiments, may have specific three dimensional structural characteristics, and or specific charge characteristics. An epitope is a region of an antigen that is bound by an antibody.

"CETP:CETP modulator complex" refers to the complexation of the CETP polypeptide with the CETP modulator as described herein. Preferably the "CETP:CETP modulator complex" results from the contact of CETP polypeptide with a compound that binds to cysteine 13 or other free cysteines, preferably that binds to cysteine 13 of the CETP polypeptide as described herein.

The term "binding" or "bind to" refers to the specific association or other specific interaction between two molecular species, such as protein-protein or protein-small molecule interactions. It is contemplated that such association is mediated through specific binding sites on each of the two interacting molecular species.

The term "binding site" refers to the reactive region or domain of a molecule that directly participates in its specific binding with another molecule. For example, when referring to the binding site on a protein, binding occurs as a result of the presence of specific amino acid sequence that interacts with the other molecule.

The term "hygroscopic polymeric excipient(s)" means polymeric excipient(s) which take(s) up moisture for example by absorption or adsorption even at relative humidity as low as 50%, at room temperature (e.g. about 25 °C). The moisture uptake is measured e.g by dynamic vapor sorption at room temperature. As an example the hygroscopicity can be measured in accordance with the method disclosed in the European Pharmacopoeia - 6th Edition (2008), Chapter 5.11. The dynamic vapor sorption technique measures the change in mass which is produced by varying the vapor concentration surrounding the product. Suitable "hygroscopic polymeric excipients" are hydroxypropyl methylcellulose, hydroxypropyl cellulose, low-substituted hydroxypropyl cellulose, hydroxyethylmethyl cellulose, carboxypolymethylene, methylcellulose, ethylcellulose, hydroxyethyl cellulose, celluloseacetate, polyvinylpyrrolidone crosslinked polyvinylpyrrolidone, micronized crosslinked polyvinylpyrrolidone, carboxymethylcellulose sodium, carboxymethylcellulose calcium, crosslinked carboxymethylcellulose, microcrystalline cellulose, silicified microcrystalline cellulose, cellulose powder, carboxymethyl starch, starch, pregelatinized starch or mixture thereof. In particular "hygroscopic polymeric excipients" refer to hydroxypropyl methylcellulose, carboxymethylcellulose sodium, microcrystalline cellulose and micronized crosslinked polyvinylpyrrolidone. Examples of "water insoluble hygroscopic polymers" at room temperature (e.g. about 25 °C) include low-substituted hydroxypropyl cellulose, carboxypolymethylene, ethylcellulose, celluloseacetate, crosslinked polyvinylpyrrolidone, micronized crosslinked polyvinylpyrrolidone, carboxymethylcellulose calcium, microcrystalline cellulose, silicified microcrystalline cellulose, cellulose powder, and starch.

The term "Super-disintegrant" refers to disintegrants that very rapidly expand upon contact with water. Generally speaking, superdisintegrants are disintegration agents which can be used in a fractional amount of normal disintegrants to obtain the same effect. Examples of superdisintegrants include cross-linked carboxymethyl cellulose sodium (a.k.a. croscarmellose sodium), sodium starch glycolate, and cross-linked polyvinyl pyrollidone (a.k.a. crospovidone). Croscarmellose sodium is commercially available from FMC Corp. under the trade name Ac-Di-Sol® and from Avebe Corp. under the trade name Primellose®®. Sodium starch glycolate is commercially available from Penwest Pharmaceuticals Co. under the tradename Explotab® and from Avebe Corp. under the tradename Primojel®. Crospovidone is commercially available from BASF Corp. under the tradename Kollidon® CL and from International Specialty Chemicals Corp. under the tradename Polyplasdone®. Croscarmellose is also commercially available from Mingtai Chemical Co. Ltd under the tradename DISOLCEL® and from J. Rettenmaier & Söhne GmbH + Co (JRS) under the tradename Vivasol®. The most preferred superdisintegrants are croscarmellose sodium and crospovidone.

The term "water instable" means the presence of a hydrolysis sensitive functional group like an ester, amide or thioester.

In a particular embodiment of the invention, the present invention shows positive interaction of a combination of a CETP modulator with carotenoid(s), more particular with xanthophyll(s) such as lutein, trans-zeaxanthin, meso-zeaxanthin and astaxanthin either alone or in combination thereof in the prevention, treatment, delaying progression and /or reduction of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD..

In a particular embodiment of the invention, the present invention shows a synergy of a combination of a CETP modulator with carotenoid(s), more particular with xanthophyll(s) such as lutein, trans-zeaxanthin, meso-zeaxanthin and astaxanthin either alone or in combination thereof in the prevention, treatment, delaying progression and /or reduction of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD..

Unless otherwise stated all percentages are given in weight percent of the total weight of the composition.

In a particular embodiment, the "CETP modulator" is thioisobutyric acid S-(2-{[1-(2-ethylbutyl)-cyclohexanecarbonyl]-amino}-phenyl) ester, also know as S-[2-([[1-(2-ethylbutyl)-cyclohexyl]-carbonyl]amino)phenyl]2-methylpropanethioate, dalcetrapib or a compound of formula I

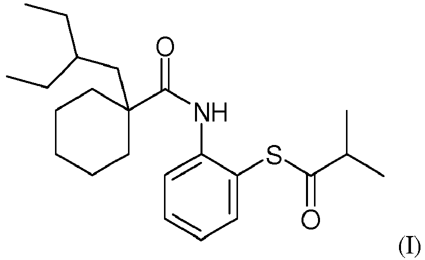

S-[2-([[1-(2-ethylbutyl)cyclohexyl] carbonyl] amino) phenyl] 2- methylpropanethioate has been shown to be a modulator of CETP activity in humans (de Grooth et al., Circulation, 105, 2159-2165 (2002)) and rabbits (Shinkai et al., J. Med. Chem., 43, 3566-3572 (2000); Kobayashi et al., Atherosclerosis, 162, 131-135 (2002); and Okamoto et al., Nature, 406 (13), 203-207 (2000)). S-[2-([[1-(2- ethylbutyl) cyclohexyl] carbonyl] amino) phenyl] 2-methylpropanethioate has been shown to increase plasma HDL cholesterol in humans (de Grooth et al., supra) and in rabbits (Shinkai et al., supra ; Kobayashi et al., supra ; Okamoto et al., supra). Moreover, S-[2-([[1-(2-ethylbutyl) cyclohexyl] carbonyl] amino) phenyl] 2-methylpropanethioate has been shown to decrease LDL cholesterol in humans (de Grooth et al. , supra) and rabbits (Okamoto et al., supra). S-[2-([[1-(2-ethylbutyl)cyclohexyl] carbonyl] amino) phenyl] 2-methylpropanethioate, as well as methods of making and using the compound, are described in EP patent EP1020439, Shinkai et al., J. Med. Chem. 43:3566-3572 (2000) or WO 2007/051714, WO 2008/074677 or WO2011/000793.

In a preferred embodiment the CETP modulator (e.g. compound of formula I) is a solid in crystalline or amorphous form, more preferably in crystalline form. In a particular embodiment S-[2-([[1-(2-ethylbutyl)-cyclohexyl]-carbonyl]amino)phenyl]2-methylpropanethioate is in crystalline form A.

Form A is characterized by an X-ray powder diffraction pattern having peaks at about 7.9°, 8.5°, 11.7°, 12.7°, 17.1°, 18.0°, 18.5°, 20.2°, 22.1°, 24.7° ± 0.2 °, particularly by an XRPD peaks observed at an angle of diffraction 2Theta of 7.9°, 11.7°, 17.1°, 18.5° (±0.2°).

While not wishing to be bound by any particular theory, it is hypothesized that within the body of a patient, Compound I is hydrolyzed in plasma, the liver, and/or the small intestine to form S-[2([[1-(2-ethylbutyl)cyclohexyl]carbonyl]amino)phenyl] thiol (herein referred to as Compound II). It is known that low molecular weight thiol components (i.e., R-SH), such as cysteine and glutathione, and high molecular weight thiol components (i.e., Prot-SH), such as peptides and proteins (e.g., enzymes and cell membranes), exist in the body as mixed disulfides containing an oxidized disulfide bond 10 (S-S bond) between or within the molecule (see, e.g., Shimade et al., J Chromatogr. B, 659, 227 (1994)). Therefore, it is hypothesized that within the body of a patient, Compound II is conjugated with low or high molecular weight thiols to yield mixed disulfides or to yield dimers of Compound II. Since these forms are in an oxidation-reduction equilibrium with each other via Compound II, all of these forms, as well as Compound II, are collectively, but not exclusively, considered and referred to hereafter as the active form of Compound I. The following scheme depicts the above-described hypothesis.

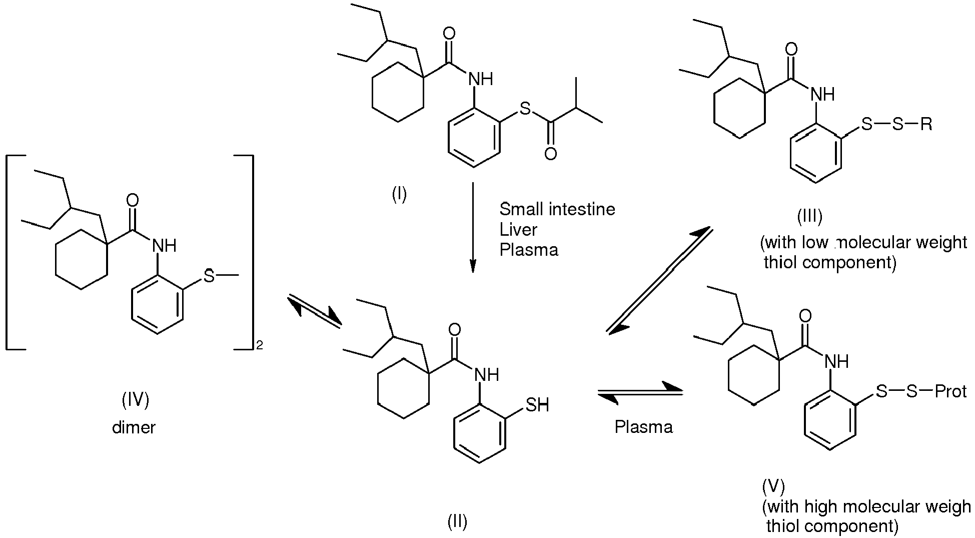

While the administration of Compound I is a particularly preferred embodiment of the invention, the invention also contemplates the administration of other compounds that will yield the active form of Compound I, i.e., other prodrugs of the active form of Compound I. Such prodrugs, for example, can be compounds that have different mercapto-protecting groups, but that still result in the formation of the active form of Compound I (e.g., Compound II) in the body of a patient (i.e., in vivo). The term "mercapto-protecting groups" refers to commonly used mercapto-protecting groups (e.g., as described in Wolman, The Chemistry of the Thiol Group, D. Patai, Ed., Wiley-Interscience, New York, 1974). Any organic residues that can be dissociated in vivo may be used without particular restriction. Examples of particularly suitable mercapto-protecting groups are described in U.S. Patent 6,426,365. The invention further contemplates the administration of Compound I' (wherein R' signifies an organic residue other than an isopropyl group) so as to yield the active form of Compound I.

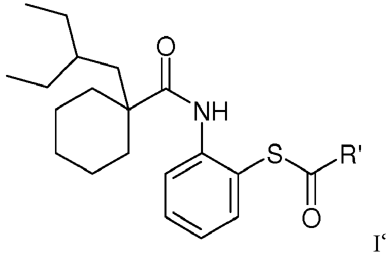

In addition, Compounds III, IV, and V (wherein R signifies an organic residue and Prot signifies a peptide or protein), which are believed to be in equilibrium with Compound II in vivo, similarly can be directly administered to the patient.

The method for assessing the change in conformation of Cholesterol ester transfer protein (CETP), can comprise:
- taking plasma sample from patient treated with a CETP modulator wherein the CETP modulator binds to an epitope within the CETP polypeptide which induces conformational changes;
- measuring the ability of an antibody to bind or not to the CETP:CETP modulator complex, wherein the antibody binds to an epitope of the CETP polypeptide wherein the said epitope is only accessible (exposed) to the antibody either in the CETP:CETP modulator complex form or in the CETP native form. In Particular the antibody can be JHC 1. JHC-1 antibody can be obtained from Japan Tobacco.

The antibody can be produced using Balb/c mice immunized against CETP that was partially purified from lipoprotein-depleted healthy human plasma by Phenyl Sepharose HP column (Amersham Pharmacia Biotech) and Resource Q column (Amersham Pharmacia Biotech) chromatographies, in accordance with the disclosure of Takahashi H, Biochem Biophys. Res. Commun 2001, 27, 283, 118.

Other antibodies were raised in Naval Medical Research Institute (NMRI) mice injected intraperitoneally with 20 µg of rhCETP emulsified in aluminum hydroxide gel (Alhydrogel-2%, Brenntag Biosector) containing CPG-OGN according to Davis et al 1998. The animals received four booster injections each at 3-week intervals with the same antigen preparation.

As soon as the animals showed a specific immune response to the recombinant human CETP (rhCETP), the best responders were boosted and after 3 days, the spleens were removed and the isolated cells fused to PAI myeloma cells, a variant of the P3-x63-AG8 myeloma (Kohler & Milstein 1975). The following monoclonal antibodies were selected for further characterization: 6/2, 6/6 6/17.

Although within the hereunder examples 6/6 was used as internal detection antibody/conjugate antibody, it could have easily been replaced by JHC2 from the above mentioned referenced. This antibody would have been produced in similar manner to the procedure disclosed in Takahashi H, Biochem Biophys. Res. Commun 2001, 27, 283, 118.
- More particularly, the method for assessing the change in conformation could be an Enzyme-linked immunosorbent assay (ELISA) method. For instance the ELISA method could be carried out as follows:
   - -apply a solution of plasma sample of a patient being treated with the CETP modulator on the antibodies, in particular JHC1 or 6/2,
   - Incubate,
   - Rinse the excess,
   - Apply a solution of the conjugated antibody, in particular JHC 2 or 6/6,
   - quench the reaction,
   - Measure optical density.

Alternatively the method of conformation change can be found in Okamoto H, Nature 2000; 406(6792):203-207.

In a particular embodiment, the invention relates to "CETP modulators" which are useful in the prevention of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD..

In another particular embodiment, the invention relates "CETP modulators" which are useful in the treatment of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In another particular embodiment, the invention relates "CETP modulators" which are useful in delaying progression of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In another particular embodiment, the invention relates "CETP modulators" which are useful in the reduction of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In another embodiment, the invention comprises a "CETP modulator" for use as therapeutic active substances for the treatment of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In another embodiment, the invention comprises a "CETP modulator" for use as therapeutic active substances for the prophylaxis of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In another embodiment, the invention comprises a "CETP modulator" for use as therapeutic active substances for delaying progression of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In another embodiment, the invention comprises a "CETP modulator" for use as therapeutic active substances for the reduction of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD. In a particular embodiment, the present invention comprises a pharmaceutical composition comprising the cholesteryl ester transfer protein modulator, such as 5-[2-([[1-(2-ethylbutyt)cyclohexyl]carbonyl]amino)phenyl] 2-methylpropanethioate and crospovidone, useful for the prevention, treatment, delaying progression, and/or reduction of eye diseases, such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In another embodiment of the present invention, the composition as described therein is a pharmaceutical composition useful in particular for the prevention of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In another embodiment of the present invention, the composition as described therein is a pharmaceutical composition useful in particular for the treatment of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In another embodiment of the present invention, the composition as described therein is a pharmaceutical composition useful in particular for delaying progression of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In another embodiment of the present invention, the composition as described therein is a pharmaceutical composition useful in particular for the reduction of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD.

In another embodiment, the invention comprises a therapeutic dose of "CETP modulator" for use as therapeutic active substances for the reduction of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD. The term "therapeutic dose" in this context means that the compound(s) produce(s) a change in the symptoms or conditions associated with the disease or condition which is being treated. It is sufficient that a therapeutic dose produce an incremental change in the symptoms or conditions associated with the disease; a cure or complete remission of symptoms is not required. One having ordinary skill in this art can easily determine whether a dose is therapeutic by establishing criteria for measuring changes in symptoms or conditions of the disease being treated and then monitoring changes in these criteria according to known methods. External physical conditions, histologic examination of affected tissues in patients or the presence or absence of specific cells or compounds, associated with a disease may provide objective criteria for evaluating therapeutic effect. In one example, methods of the invention may be used to treat AMD where therapeutic effect is assessed by changes in preventing vision loss. Other indicators of therapeutic effect will be readily apparent to one having ordinary skill in the art and may be used to establish efficacy of the dose. See also section entitled herein, "Efficacy of the Treatment."

The doses may be administered according to any time schedule which is appropriate for treatment of the disease or condition. For example, the dosages may be administered on a daily, weekly, biweekly or monthly basis in order to achieve the desired therapeutic effect and reduction in adverse effects. The dosages can be administered before, during or after the development of the disorder. The specific time schedule can be readily determined by a physician having ordinary skill in administering the therapeutic compound by routine adjustments of the dosing schedule within the method of the present invention. The time of administration of the number of first individual and second individual doses as well as subsequent dosages is adjusted to minimize adverse effects while maintaining a maximum therapeutic effect. The occurrence of adverse effects can be monitored by routine patient interviews and adjusted to minimize the occurrence of side effects by adjusting the time of the dosing. For example, doses may be administered on a daily schedule.

CETP modulator, which are useful in the prevention, treatment, delaying progression and /or reduction of eye diseases such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD, can also be used in association with other active components. For example, compounds of formula (I) can be used in association with anti VEGF compounds.

In the context of the present specification, by "in association with" it should be understood a coadministration, or a combination of two active principles. The coadministration can be simultaneous, almost simultaneous, or delayed in time by a few days or weeks, for example by up to 4 or 5 weeks.

Vascular endothelial growth factor (VEGF) is an endogenous molecule involved in a number of physiological processes, including blood vessel growth at the foetal stage, during injury healing, or for the growth of new vessels in tissues that have a deficient blood supply. VEGF is also involved in pathological processes, like the development of tumour blood vasculature which allows for growth and spread of the tumour, or the formation of new blood vessels in the eye that eventually contributes to vision loss. AntiVEGF therapies therefore aim to prevent this abnormal blood vessel formation by blocking VEGF action.

Examples of anti-VEGF compounds include Macugen® (Pegaptanib sodium), Lucentis (ranibizumab), Avastatin® (bevacizumab) RhuFab, or VEGF Trap Eye. Therefore, in another aspect, CETP modulator is used in association with antiVEGF compounds for the prevention, treatment and/ or reduction of age-related macular degeneration. In another embodiment, the present invention provides a CETP modulator and one or more carotenoids, in particular wherein the carotenoid are xanthophylls, more particularly wherein the carotenoid are lutein with optionally one stereoisomer of zeaxanthin for the prevention, the treatment, delaying progression and /or the reduction of eye diseases, such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, age-related macular degeneration (AMD), more particularly AMD, most particularly dry AMD..

In an embodiment, at least one zeaxanthin isomer is selected from the group consisting of zeaxanthin and meso-zeaxanthine. In a further embodiment, the amount of lutein ranges from 0.5 to 25 mg, and the amount of at least one zeaxanthin isomer ranges from 0.1-5 mg. Advantageously, lutein and at least one zeaxanthin isomer are present in the composition in a ratio lutein:zeaxanthin isomer of about 5: 1. In a preferred embodiment, the association is an intimate mixture of a CETP modulator, lutein and at least one zeaxanthin isomer.

The "CETP modulator" or the composition according to the present invention is for treating mammal (i.e.cat, dog, hamster, rabbit, mouse, gerbil, rat, Guinea pig, or human, especially a human (i. e. a male or female human).

Accordingly, the invention provides a method for the treatment or prophylaxis of eye diseases in a mammal, which method comprises administering to a mammal (preferably a mammal in need thereof) a therapeutically effective amount of the pharmaceutical composition. The mammal preferably is a human (i. e. , a male or female human). The human can be of any race (e. g. , Caucasian or Oriental). The eye diseases is preferably selected from degenerative disease or damage to the retina caused by a disease or an injury, eye strain, accommodative dysfunction of the eye, asthenopia, diabetic retinopathy or dry eye syndrome, the latter caused by either tear or oil gland inflammation. In particular, the method comprises administering a therapeutically effective amount of the composition to an individual to benefit the vision of an individual suffering from eye damage caused by disease or injury or to prevent such disease in man. Most particularly the eye diseases are selected from cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization or age-related macular degeneration (AMD), more particularly AMD, most particularly dry AMD.

In certain embodiment of the present invention the composition comprises: 10% to 69% by weight, preferably 40% to 60% by weight, more preferably 48% to 55% by weight of CETP modulator.

In certain embodiments of the present invention, the composition further comprises: 1% to 10% by weight, preferably 5% to 10% by weight, more preferably 4% to 8% by weight of a super-disintegrant.

In certain embodiments of the present invention as defined herein, the super-disintegrant is a hygroscopic polymeric excipient. In particular the hygroscopic polymeric excipient as superdisintegrant is croscarmellose sodium.

In a particular embodiment, the present invention provides a composition comprising:
a)S-[2-([[1-(2-ethylbutyl)-cyclohexyl]-carbonyl]amino)phenyl]2-methylpropanethioate,
b) crospovidone micronized and
c) croscarmellose sodium;
useful for the prevention, treatment, delaying progression, and/or reduction of eye diseases, such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization or age-related macular degeneration (AMD), more particularly AMD, most particularly dry AMD..

In certain embodiments of the present invention as defined herein, the composition comprises 10% to 69% by weight of S-[2-([[1-(2-ethylbutyl)-cyclohexyl]-carbonyl]amino)phenyl]2-methylpropanethioate or a prodrug compound thereof.

In certain embodiments of the present invention, the pharmaceutical composition comprises: 10% to 69% by weight, preferably 40% to 60% by weight, more preferably 48% to 55% by weight of S-[2-([[1-(2-ethylbutyl)-cyclohexyl]-carbonyl]amino)phenyl]2-methylpropanethioate or a prodrug compound thereof.

In certain embodiments of the present invention as defined herein, the composition comprises 1% to 10% by weight, preferably 5% to 10% by weight, more preferably 5% to 8% by weight of croscarmellose sodium. More particularly, in a certain embodiment, the composition comprises 5% to 7% by weight of croscarmellose sodium.

In a particular embodiment, the composition comprises:
- 10% to 69% by weight, preferably 40% to 60% by weight, more preferably 48% to 55% by weight of S-[2-([[1-(2-ethylbutyl)-cyclohexyl]-carbonyl]amino)phenyl]2-methylpropanethioate;
- 1% to 10% by weight, preferably 5% to 10% by weight, more preferably 4% to 8% by weight of croscarmellose sodium, and
- 30% to 90% by weight, preferably 34% to 44% by weight, more preferably 40% to 44% by weight of the hygroscopic polymeric excipients;
wherein the hygroscopic polymeric excipients are selected from hydroxypropylmethyl cellulose, microcrystalline cellulose and micronized crosslinked polyvinylpyrrolidone
In certain embodiments of the present invention as defined herein, the composition comprises:
a) 48% to 55% by weight of S-[2-([[1-(2-ethylbutyl)-cyclohexyl]-carbonyl]amino)phenyl]2-methylpropanethioate;
b) 4% to 8% by weight, of croscarmellose sodium
c) 32% to 41% by weight of water insoluble hygroscopic polymer; and
d) 4% to 5% by weight of water soluble hygroscopic polymer.

In certain embodiments of the present invention as defined herein, wherein the hygroscopic polymeric excipients are selected from hydroxypropylmethyl cellulose, hydroxypropyl cellulose, low-substituted hydroxypropyl cellulose, hydroxyethylmethyl cellulose, carboxypolymethylene, methylcellulose, ethylcellulose, hydroxyethyl cellulose, celluloseacetate, polyvinylpyrrolidone, crosslinked polyvinylpyrrolidone, micronized crosslinked polyvinylpyrrolidone, carboxymethylcellulose calcium, crosslinked carboxymethylcellulose, microcrystalline cellulose, silicified microcrystalline cellulose, cellulose powder, carboxymethyl starch, starch and pregelatinized starch.

In certain embodiments of the present invention as defined herein, wherein the hygroscopic polymeric excipients are hydroxypropylmethyl cellulose, microcrystalline cellulose and micronized crosslinked polyvinylpyrrolidone.

The pharmaceutical composition can be prepared by any suitable method, such as those methods well known in the art of pharmacy, for example, methods such as those described in Gennaro et aI., Remington's Pharmaceutical Sciences (18th ed., Mack Publishing Co., 1990), especially Part 8: Pharmaceutical Preparations and their Manufacture. Such methods include the step of bringing into association the CETP modulator with the other components of the pharmaceutical composition. In particular, the composition of the present invention may be prepared according to any known process which results in keeping the API substantially in crystalline form (the amount of the hydrophobic, API in amorphous does not exceed 10% by weight). Furthermore, the composition of the present invention may be prepared according to any known process which results in keeping the API substantially in crystalline form (the amount of the hydrophobic, water instable compound with a waxy consistency substantially in amorphous does not exceed 10% by weight). The composition, in particular the pharmaceutical composition can be prepared according to WO2004/082593 and WO2012/059447.

In certain embodiments of the present invention as defined herein, the two diluents are hygroscopic polymeric excipients. In particular the hygroscopic polymeric excipients as diluents are ethylcellulose, micronized crosslinked polyvinylpyrrolidone, microcrystalline cellulose, silicified microcrystalline cellulose, cellulose powder, starch, pregelatinized starch.

In certain embodiments of the present invention as defined herein, at least two hygroscopic polymeric excipients are present.

In certain embodiments of the present invention as defined herein, the super-disintegrant and at least one of the diluents, or at least two diluents are hygroscopic polymeric excipients. More preferably, at least the super-disintegrant and one of the diluents are hygroscopic polymeric excipients.

In certain embodiments of the present invention as defined herein, the super-disintegrant and the two diluents are hygroscopic polymeric excipients.

In certain embodiments of the present invention as defined herein, there is at least 30% by weight of hygroscopic polymeric excipients, preferably 44% to 50% by weight.

In certain embodiments of the present invention, the super-disintegrant is croscarmellose sodium. In particular, the present invention comprises up to 6 % by weight of croscarmellose sodium.

The invention provides a physically stable pharmaceutical composition comprising at least one hydrophobic and water instable cholesteryl ester transfer protein (CETP) modulator or a combination thereof embedded in a chemically protective hygroscopic polymer matrix tablet consisting of at least one hygroscopic polymer e.g. hydroxypropylmethyl cellulose (HPMC), hydroxypropyl cellulose (HPC), low-substituted hydroxypropyl cellulose (L-HPC), hydroxyethylmethyl cellulose (HEMC), carboxypolymethylene (Carbomer), methylcellulose (MC), ethylcellulose (EC), hydroxyethyl cellulose (HEC), celluloseacetate, polyvinylpyrrolidone(PVP), crosslinked polyvinylpyrrolidone (Crospovidone), micronized crosslinked polyvinylpyrrolidone (crospovidone micronized), carboxymethylcellulose sodium (croscarmellose sodium, CMC Na), carboxymethylcellulose calcium (croscarmellose calcium, CMC Ca), crosslinked carboxymethylcellulose (Crosslinked CMC), microcrystalline cellulose (MCC), silicified microcrystalline cellulose (silicified MCC), cellulose powder, carboxymethyl starch (sodium starch glycolate), starch (maize starch, potato starch, rize starch, wheat starch, tapioca starch), pregelatinized starch or a combination thereof in an amount of preferably 40% by weight or more per unit.

According to the present invention the composition comprises at least S-[2-([[1-(2-ethylbutyl)-cyclohexyl]-carbonyl]amino)phenyl]2-methylpropanethioate or prodrug compound thereof or a combination thereof embedded in a chemically protective hygroscopic polymer matrix tablet consisting of at least one hygroscopic polymer e.g. hydroxypropylmethyl cellulose (HPMC), hydroxypropyl cellulose (HPC), low-substituted hydroxypropyl cellulose (L-HPC), hydroxyethylmethyl cellulose (HEMC), carboxypolymethylene (Carbomer), methylcellulose (MC), ethylcellulose (EC), hydroxyethyl cellulose (HEC), celluloseacetate, polyvinylpyrrolidone(PVP), crosslinked polyvinylpyrrolidone (Crospovidone), micronized crosslinked polyvinylpyrrolidone (crospovidone micronized), carboxymethylcellulose sodium (croscarmellose sodium, CMC Na), carboxymethylcellulose calcium (croscarmellose calcium, CMC Ca), crosslinked carboxymethylcellulose (Crosslinked CMC), microcrystalline cellulose (MCC), silicified microcrystalline cellulose (silicified MCC), cellulose powder, carboxymethyl starch (sodium starch glycolate), starch (maize starch, potato starch, rize starch, wheat starch, tapioca starch), pregelatinized starch or a combination thereof in an amount of preferably 40% by weight or more per unit.

In particular according to the present invention the composition comprises at least one hydrophobic and water instable cholesteryl ester transfer protein (CETP) modulator embedded in a chemically protective hygroscopic polymer matrix tablet consisting of hydroxypropylmethyl cellulose, carboxymethylcellulose sodium, microcrystalline cellulose and micronized crosslinked polyvinylpyrrolidone.

In particular according to the present invention the composition comprises at least S-[2-([[1-(2-ethylbutyl)-cyclohexyl]-carbonyl]amino)phenyl]2-methylpropanethioate embedded in a chemically protective hygroscopic polymer matrix tablet consisting of hydroxypropylmethyl cellulose, carboxymethylcellulose sodium, microcrystalline cellulose and micronized crosslinked polyvinylpyrrolidone.

In particular embodiment, the composition comprises at least S-[2-([[1-(2-ethylbutyl)-cyclohexyl]-carbonyl]amino)phenyl]2-methylpropanethioate embedded in a chemically protective hygroscopic polymer matrix tablet consisting of hydroxypropylmethyl cellulose, carboxymethylcellulose sodium, microcrystalline cellulose and micronized crosslinked polyvinylpyrrolidone in an amount of preferably 40% by weight or more per unit.

In another embodiment of the present invention provides the composition comprises:
a) 48% to 55% by weight of S-[2-([[1-(2-ethylbutyl)-cyclohexyl]-carbonyl]amino)phenyl]2-methylpropanethioate;
b) less than 12% by weight of crospovidone micronized; and
c) 35% to 44% by weight of hydroxypropylmethyl cellulose, microcrystalline cellulose and croscarmellose sodium.

In another embodiment of the present invention provides the composition comprises:
a) S-[2-([[1-(2-ethylbutyl)-cyclohexyl]-carbonyl]amino)phenyl]2-methylpropanethioate;
b) microcrystalline cellulose;
c) crospovidone micronized;
d) hydroxypropylmethyl cellulose; and
e) croscarmellose Sodium.

In another embodiment of the present invention provides the composition comprises:
a) 48% to 55% by weight of S-[2-([[1-(2-ethylbutyl)-cyclohexyl]-carbonyl]amino)phenyl]2-ethylpropanethioate;
b) 24% to 26% by weight of microcrystalline cellulose;
c) 11% to 12% by weight of crospovidone micronized;
d) 4% to 5% by weight of hydroxypropylmethyl cellulose;
e) 4% to 6 % by weight of croscarmellose sodium.

In another embodiment of the present invention provides the composition comprises:
a) 48% to 55% by weight of S-[2-([[1-(2-ethylbutyl)-cyclohexyl]-carbonyl]amino)phenyl]2-ethylpropanethioate;
b) 24% to 26% by weight of microcrystalline cellulose;
c) 11% to 12% by weight of crospovidone micronized;
d) 4% to 5% by weight of hydroxypropylmethyl cellulose;
e) 4% to 6 % by weight of croscarmellose sodium;
f) 0 to 1% by weight of magnesium stearate;
g) 0 to 1% by weight of colloidal silicon dioxide;
h) 0 to 1% by weight of sodium stearyl fumarate.

In particular embodiments of the present invention, the compositions described herein are pharmaceutical compositions.

The pharmaceutical composition can be, for example, in the form of a pill, capsule or tablet, each containing a predetermined amount of CETP modulator and in particular coated for ease of swallowing, in the form of a powder or granules. In particular, the pharmaceutical composition is in the form of a tablet comprising the CETP modulator and the components of the tablet utilized and described therein. For oral administration, fine powders or granules may contain diluting, dispersing and/or surface active agents and may be present, for example, in capsules or sachets in the dry state, or in tablets wherein binders and lubricants may be included. Components such as sweeteners, flavoring agents, preservatives, suspending agents, thickening agents, and/or emulsifying agents also may be present in the pharmaceutical composition.

In a particular embodiment, the composition herein is filmed coated, with polyvinyl alcohol based coat (PVA-based coat), particularly with 20 mg or less PVA-based coat, more particularly with 15 mg PVA-based coat.

In certain embodiments of the present invention, the composition comprises 100 mg to 600 mg of S-[2-([[1-(2-ethylbutyl)-cyclohexyl]-carbonyl]amino)phenyl]2-methylpropanethioate. In particular, the composition comprises 150 mg to 450 mg of S-[2-([[1-(2-ethylbutyl)-cyclohexyl]-carbonyl]amino)phenyl]2-methylpropanethioate. More particularly, the composition comprises 250 mg to 350 mg of S-[2-([[1-(2-ethylbutyl)-cyclohexyl]-carbonyl]amino)phenyl]2-methylpropanethioate. Most particularly, the composition comprises 250 mg to 350 mg of S-[2-([[1-(2-ethylbutyl)-cyclohexyl]-carbonyl]amino)phenyl]2-methylpropanethioate.

In another embodiment, the composition comprises for pediatric use 25mg to 300mg of S-[2-([[1-(2-ethylbutyl)-cyclohexyl]-carbonyl]amino)phenyl]2-methylpropanethioate. In particular the peaditric composition comprises 75mg to 150mg of S-[2-([[1-(2-ethylbutyl)-cyclohexyl]-carbonyl]amino)phenyl]2-methylpropanethioate.

To be effective, CETP modulator must be absorbed into the blood. Oral dosing of CETP modulator is preferred because to be effective such "CETP modulators" must be taken on a regular basis, such as daily.

The CETP modulator can be administered to the mammal at any suitable dosage (e. g. , to achieve a therapeutically effective amount). For example, a suitable dose of a therapeutically effective amount of compound of formula I for administration to a patient will be between approximately 100 mg to about 1800 mg per day. In particular, a desirable dose is about 300 mg to about 900 mg per day. A preferred dose is about 600 mg per day.

In another embodiment the invention provides a kit comprising a CETP modulator as described herein, lutein and optionally with one or more stereoisomer of zeaxanthin. In a more particular embodiment the invention provides a kit comprising a CETP modulator as described herein, lutein and optionally with one or more stereoisomer of zeaxanthin., prescribing information also known as "leaflet", a blister package or bottle (HDPE or glass) and a container. The prescribing information preferably includes the advice to a patient regarding the administration of the CETP modulator (e.g. compound of formula (I) with food, especially to improve the bioavailability of the CETP modulator.

In another embodiment the invention provides a kit comprising a composition as described herein, lutein and optionally with one or more stereoisomer of zeaxathin. In a more particular embodiment the invention provides a kit comprising a composition as described herein, lutein and optionally with one or more stereoisomer of zeaxathin, prescribing information also known as "leaflet", a blister package or bottle (HDPE or glass) and a container. The prescribing information preferably includes the advice to a patient regarding the administration of the CETP modulator (e.g. compound of formula (I) with food, especially to improve the bioavailability of the CETP modulator.

In another embodiment, the invention provides a kit comprising a composition comprising a therapeutically effective amount of S-[2-([[1-(2-ethylbutyl)-cyclohexyl]-carbonyl]amino)phenyl]2-methylpropanethioate and at least 30% by weight of hygroscopic polymeric excipients by composition weight, lutein and optionally with one or more stereoisomer of zeaxanthin., prescribing information, a blister package or bottle and a container. In particular embodiment the invention provides the kit as described herein, wherein the prescribing information includes the advice to a patient regarding the administration of S-[2-([[1-(2-ethylbutyl)-cyclohexyl]-carbonyl]amino)phenyl]2-methylpropanethioate with food.

In another embodiment, the invention provides a tablet comprising the composition as herein described.

In another embodiment, the invention provides a composition as herein described for preparing a medicament for the treatment or prevention of eye diseases, such as cataract, corneal clouding (opacification), glaucoma, uveitis, intraocular neovascular diseases, in particular proliferative retinopathies, choroidal neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization or age-related macular degeneration (AMD), more particularly AMD, most particularly dry AMD, in particular wherein the S-[2-([[1-(2-ethylbutyl)-cyclohexyl]-carbonyl]amino)phenyl]2-methylpropanethioate is administered at a daily dose of 100mg to 1800mg, particularly 300mg to 900mg, more particularly 600mg, more particularly wherein S-[2-([[1-(2-ethylbutyl)-cyclohexyl]-carbonyl]amino)phenyl]2-methylpropane thioate is administered with food.

Other features and embodiments of the invention will become apparent from the following examples which are given for illustration of the invention rather than for limiting its intended scope.

### Example A:

XRPD patterns of S-[2-([[1-(2-ethylbutyl)cyclohexyl] carbonyl] amino) phenyl] 2-methylpropanethioate crystalline form A were recorded at ambient conditions in transmission geometry with a STOE STADI P diffractometer (Cu K alpha radiation source, primary monochromator, position sensitive detector, angular range 3° to 42° 2Theta, approximately 60 minutes total measurement time). The samples were prepared and analyzed without further processing (e.g. grinding or sieving) of the substance.

| 2theta / ° | relative intensity / % |
|---|---|
| 7.9 | 86.3 |
| 8.5 | 16.2 |
| 11.7 | 30.7 |
| 12.7 | 17.1 |
| 17.1 | 41.6 |
| 18 | 14.6 |
| 18.5 | 100 |
| 20.2 | 27.2 |
| 22.1 | 33.7 |
| 24.7 | 11.9 |

### Example 1: Golden Syrian hamsters study a:

### MATERIAL AND METHODS

Hamsters (7 weeks-old) were fed a standard rodent chow diet containing 4.5% fat and 0.05% cholesterol and coconut oil (70 g/kg dry food) for 14 days. After two weeks of feeding, hamsters were allocated to 2 experimental groups of 10 animals and given the same diet as before supplemented with 0.1% (w:w) FLORAGLO (lutein 5.2 %, zeaxanthin 0.5%, DSM Nutritional Products): group 1 received no treatment, group 2 was given dalcetrapib as a 0.375% food admixture (-400 mg/kg).

After 14 days of treatment and 4 hours of fasting, plasma, liver and eyes were collected and kept frozen until analysis. Lutein and zeaxanthin levels in plasma, liver and the pooled eyecups from 2 hamsters were determined by HPLC-MS using cryptoxanthin as internal standard. Plasma samples were directly extracted with hexane/ethyl acetate in dark environment in the presence of BHT. Liver samples and pooled eye cups (n=4) were first homogenized in the presence of BHT using an appropriate solvent in a Precellys tissue homogenizer followed by saponification prior extraction with hexane/ethyl acetate. The organic phase was washed with water. All organic phases were dried and reconstituted in methanol containing 0.1%. HPLC-MS was accomplished on a Waters TQ-S in APCI mode coupled to an Acquity I-class UPLC (Waters AG, Baden-Dättwil) on an ACCLAIM C30 RP column (Thermo Fisher Scientific, Reinach).

### Results

Change in plasma, liver and eyecups following dalcetrapib treatment.

| | Lutein | Zeaxanthin |
|---|---|---|
| Plasma | + 55.7 | + 164 |
| *p* | ** | *** |
| Liver | + 107 | + 222.9 |
| *p* | *** | *** |
| Eyecups | + 164.6 | + 114 |
| *p* | *** | *** |

| | | |
|---|---|---|
| In % vs control; Wilcoxon / Kruskal-Wallis's test * *p<* 0.05; ** : *p* < 0.01; *** : *p<* 0.001 Dalcetrapib increased significantly plasma, liver and eyecup lutein levels by +55.7% p<0.0019, +107% p< and +165% p<0.009 and zeaxanthin levels by +164% p< 0.0002, +222.9 p< 0.0002 and +114 % p< 0.0088. | | |

### Example 2: Golden Syrian hamsters study b:

### MATERIAL AND METHODS

Hamsters (7 weeks-old) were fed a standard rodent chow diet containing 4.5% fat and 0.05% cholesterol and coconut oil (70 g/kg dry food) for 14 days. After 2 weeks of high-fat diet feeding, hamsters were allocated to 3 experimental treatment groups of 10 animals each based on plasma lipid levels, and provided the same diet as before supplemented with 0.1% (w/w) FLORAGLO (same composition as for Study 2) and either no treatment (group 1), dalcetrapib at 0.320% (w:w, ∼300 mg/kg, group 2), or anacetrapib at 0.024% (w:w, ∼25 mg/kg, group 3).

After 14 days of treatment, plasma and livers were collected for lutein and zeaxanthin analysis as described in study a.

### Results

Change in plasma and liver following dalcetrapib and anacetrapib treatment.

| | dalcetrapib | | anacetrapib | |
|---|---|---|---|---|
| | Lutein | zeaxanthin | Lutein | zeaxanthin |
| Plasma | + 95 | + 101 | ns | ns |
| *p* | *** | *** | | |
| Liver | + 90 | + 109 | -32 | -31 |
| *p* | ** | *** | * | * |

| | | | | |
|---|---|---|---|---|
| In % vs control; Wilcoxon / Kruskal-Wallis's test * *p<* 0.05; ** : *p* < 0.01; *** : *p<* 0.001 | | | | |

It can be concluded form both in vivo studies that dalcetrapib treatment increases the intestinal uptake and tissue distribution of the protective antioxidant dietary xanthophylls such as lutein and zeaxanthin.

Additional variable measured.

| | Dalcetrapib | Anacetrapib |
|---|---|---|
| Total cholesterol | ns | +28 ** |
| HDL-C | ns | +54 *** |
| VLDL-C | -27 * | ns |
| LDL-C | ns | -99 *** |
| Triglycerides | -37 ** | ns |

| | | |
|---|---|---|
| In % vs control; Wilcoxon / Kruskal-Wallis's test * : *p<* 0.05; ** : *p* < 0.01; *** : *p*< 0.001 | | |

### Embodiments:

a) A CETP modulator for use in the prevention, treatment, delaying progression and /or reduction of eye disease..
b) A CETP modulator according to embodiment a) for use in the treatment of eye disease.
c) A CETP modulator according to any one of claim a) or b), wherein the eye disease is cataract, corneal clouding (opacification), glaucoma, uveitis or intraocular neovascular diseases.
d) A CETP modulator according to any one of embodiments a) to c), wherein the eye disease is proliferative retinopathies, Choroidal Neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization or age-related macular degeneration (AMD), more particularly AMD, most particularly dry AMD
e) A CETP modulator according to any one of embodiments a) to d) in association with an anti-VEGF compound.
f) A CETP modulator according to any one of embodiments a) to e) wherein the anti-VEGF compound is Macugen, Lucentis or Avastatin.
g) A CETP modulator according to any one of embodiments a) to f), in association with one or more carotenoids.
h) A CETP modulator according to any one of embodiments a) to g), wherein the carotenoids are xanthophylls.
i) A CETP modulator according to any one of embodiments a) to h), in association with lutein or with one stereoisomer of zeaxanthin or a mixture thereof.
j) A CETP modulator according to any one of embodiments a) to i), in association with lutein optionally with one stereoisomer of zeaxanthin.
k) A CETP modulator according to any one of embodiments a) to j) wherein the CETP modulator is 5-[2-([[1-(2-ethylbutyt)cyclohexyl]carbonyl]amino)phenyl] 2-methylpropanethioate.
1) A CETP modulator according to any one of embodiments a) to k), wherein the CETP modulator is a prodrug that forms S-[2-([[1-(2-ethylbutyl)cyclohexyl]carbonyl]amino)phenyl] thiol *in vivo*
m) A method of preventing, retarding and ameliorating eye disease which comprises administering a CETP modulator.
n) A method for treating eye disease according to embodiment m) which comprises administering a CETP modulator.
o) A method according to any one of embodiments m) to n), wherein the eye disease is cataract, corneal clouding (opacification), glaucoma, uveitis or intraocular neovascular diseases.
p) A method according to any one of embodiments m) to n), wherein the eye disease is proliferative retinopathies, Choroidal Neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization or age-related macular degeneration (AMD), more particularly AMD, most particularly dry AMD.
q) A method according to any one of embodiments m) to o), which further comprises administering an anti-VEGF compound.
r) A method according to any one of embodiments m) to q) wherein the anti-VEGF compound is Macugen, Lucentis or Avastatin.
s) A method according to any one of embodiments m) to r), which further comprises administering one or more carotenoids, in particular with one or more xanthophylls, more particularly with lutein optionally with one stereoisomer of zeaxanthin.
t) A method according to any one of embodiments m) to s), wherein the CETP modulator is 5-[2-([[1-(2-ethylbutyt)cyclohexyl]carbonyl]amino)phenyl] 2-methylpropanethioate.
u) A method according to any one of embodiments m) to t), wherein the CETP modulator is a prodrug that forms S-[2-([[1-(2-ethylbutyl)cyclohexyl]carbonyl]amino)phenyl] thiol *in vivo.*
v) A pharmaceutical composition comprising 5-[2-([[1-(2-ethylbutyt)cyclohexyl]carbonyl]amino)phenyl] 2-methylpropanethioate or the prodrug compound thereof and crospovidone, useful for the prevention, treatment, delaying progression, and/or reduction of eye diseases.
w) A pharmaceutical composition according to claim v, which further comprises one or more carotenoids.
x) A pharmaceutical composition according to any one of embodiments v to w, wherein the carotenoids are xanthophylls.
y) A pharmaceutical composition according to claim v, which further comprises lutein or with one stereoisomer of zeaxanthin or a mixture thereof.
z) A pharmaceutical composition according to any one of embodiments v to y, which further comprises an anti-VEGF compound.
aa) A pharmaceutical composition according to embodiment z), wherein the anti-VEGF compound is Macugen, Lucentis or Avastatin.
bb) A pharmaceutical composition according to any one of embodiments v) to aa), wherein the eye disease is proliferative retinopathies, Choroidal Neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization or age-related macular degeneration (AMD), more particularly AMD, most particularly dry AMD.
cc) A kit comprising a CETP modulator according to any one of embodiments a) to g), lutein and optionally with one or more stereoisomer of zeaxanthin.
dd) A kit according to claim cc), which further comprises prescribing information also known as "leaflet", a blister package or bottle (HDPE or glass) and a container.
ee) A kit according to any one of embodiments cc) to dd) wherein the eyes disease is proliferative retinopathies, Choroidal Neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization or age-related macular degeneration (AMD), more particularly AMD, most particularly dry AMD..
ff) A kit according to any one of embodiments cc) to ee) wherein the eyes disease is age-related macular degeneration.

## Claims

1. A CETP modulator for use in the treatment of eye disease, , wherein the CETP modulator is S-[2-([[1-(2-ethylbutyt)cyclohexyl]carbonyl]amino)phenyl] 2-methylpropanethioate.

2. A CETP modulator according to claim 1, wherein the eye disease is cataract, corneal clouding (opacification), glaucoma, uveitis or intraocular neovascular diseases.

3. A CETP modulator according to any one of claims 1 to 2, wherein the eye disease is proliferative retinopathies, Choroidal Neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization or age-related macular degeneration (AMD), more particularly AMD, most particularly dry AMD

4. A CETP modulator according to any one of claims 1 to 3 in association with an anti-VEGF compound.

5. A CETP modulator according to any one of claims 1 to 4, in association with lutein or with one stereoisomer of zeaxanthin or a mixture thereof.

6. A CETP modulator according to any one of claims 1 to 5, in association with lutein optionally with one stereoisomer of zeaxanthin.

7. A pharmaceutical composition comprising 5-[2-([[1-(2-ethylbutyt)cyclohexyl]carbonyl]amino)phenyl] 2-methylpropanethioate or the prodrug compound thereof and crospovidone, useful for the prevention, treatment, delaying progression, and/or reduction of eye diseases.

8. A pharmaceutical composition according to claim 7, which further comprises one or more carotenoids.

9. A pharmaceutical composition according to any one of claims 7 to 8, wherein the carotenoids are xanthophylls.

10. A pharmaceutical composition according to claim 7, which further comprises lutein or with one stereoisomer of zeaxanthin or a mixture thereof.

11. A pharmaceutical composition according to any one of claims 7 to 10, which further comprises an anti-VEGF compound.

12. A pharmaceutical composition according to any one of claims 7 to 11, wherein the anti-VEGF compound is Macugen, Lucentis or Avastatin.

13. A pharmaceutical composition according to any one of claims 7 to 12, wherein the eye disease is proliferative retinopathies, Choroidal Neovascularization (CNV), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization or age-related macular degeneration (AMD), more particularly AMD, most particularly dry AMD.
